# EUROPÄISCHE PATENTANMELDUNG

(11) **EP 0 904 804 A2**
(43) Veröffentlichungstag der Anmeldung: **31.03.1999**
(21) Anmeldenummer: 98118046.6
(22) Anmeldetag: 23.09.1998
(51) Int. Cl.: A61N 5/06

(54) **Vorrichtung zur Verstärkung elektromagnetischer Schwingungen zur Beeinflussung eines biologischen Systems**

(30) Priorität: 25.09.1997 DE 19742262; 03.09.1998 DE 19840173
(71) Anmelder: Ruschke, Thomas, 24340 Eckernförde (DE)
(72) Erfinder: Ruschke, Thomas, 24340 Eckernförde (DE)
(74) Vertreter: Sartorius, Peter, Dipl.-Ing.

(57) **Zusammenfassung**

**Die Erfindung bezieht sich auf** eine Vorrichtung 4 zur kohärenten Verstärkung elektromagnetischer Schwingungen durch induzierte Emission mittels eines Festkörpers, mit Neodym dotierter Kristalle oder Gläser, Halbleiter-Dioden oder Flüssigkeiten oder Gase, wobei die aus der Vorrichtung austretenden elektromagnetischen Schwingungen über ein Kabel oder Glasfaserkabel 2 zur Beeinflussung auf ein biologisches System, insbesondere auf einen menschlichen oder Tierkörper 11, geleitet werden. Das Kabel 2 weist eine Auslaßöffnung 7 auf, und die aus dem Kabel 2 austretenden Schwingungen oder Strahlen werden über einen Festkörper, ein Gemisch oder eine flüssige oder gasförmige Lösung 8 zur Beeinflussung auf ein biologisches System 11 geleitet, wobei der Festkörper, das Gemisch, die flüssigen oder die gasförmigen Lösungen mineralische, pflanzliche, tierische, menschliche Extrakte bzw. Produkte oder Giftstoffe als Beimischungen enthalten.

## Beschreibung

**Die Erfindung bezieht sich auf** eine Vorrichtung zur Verstärkung elektromagnetischer Schwingungen zur Beeinflussung eines biologischen Systems.

**Es sind bereits** Vorrichtungen zur kohärenten Verstärkung elektromagnetischer Schwingungen durch induzierte Emission mittels eines Festkörpers bekannt, die in der Medizin eingesetzt werden. Bei den bekannten Lasern besteht ein Interesse an der hohen Impulsleistung und Bündelung der Laserstrahlen. Zur Zeit werden beim Menschen nur Retina-Ablösungen und spezielle Arten des Gehirnkrebses mit Laserstrahlen behandelt. Die Behandlung der Netzhautablösung ist einfach, und der Patient muß nicht in Narkose gehalten werden. Nach den bisher durchgeführten Operationen tritt bei dem operierten Patienten kein Schmerz auf, und der Patient fühlt sich lediglich eine Zeitlang geblendet, was auf die Wirkung des Laserlichts und auf chemische Vorgänge auf der Retina oder im Sehnerv zurückgeht. Bei der Krebsbehandlung im Gehirn mittels Laserstrahlen wird die ausgewählte Stelle vom Laserstrahl getroffen und eine Verdampfung des Gewebes herbeigeführt.

**Demgegenüber liegt der Erfindung die Aufgabe zugrunde,** die Vorrichtung zur kohärenten Verstärkung elektromagnetischer Schwingungen durch induzierte Emission gegenüber den bekannten Lasereinrichtungen zu verbessern und ein biologisches System positiv zu beeinflussen und/oder die Heilbehandlung, beispielsweise die Behandlung von Wunden, Brandwunden, Knochenbrüchen, Tumoren, Prellungen oder Blutergüssen, wesentlich zu beschleunigen.

**Gelöst wird die Aufgabe erfindungsgemäß dadurch,** daß
a) die Vorrichtung ein Aufnahme- und/oder Wiedergabegerät für ein analoges oder digitales Tonsignal und/oder ein Speichergerät für ein analoges oder digitales Tonsignal aufweist,
b) der Ausgang zumindest eines dieser Geräte das Tonsignal an einen mit diesem verbundenen Tonsignalaufnehmer oder Wandler weitergibt,
c) der Tonsignalaufnehmer oder Wandler mit einer Vorrichtung zur kohärenten Verstärkung elektromagnetischer Schwingungen bzw. einer Vorrichtung zur Verstärkung elektromagnetischer Schwingungen oder einer Laservorrichtung wirkungsmäßig verbunden ist,
d) die aus der Laservorrichtung austretenden elektromagnetischen Schwingungen über ein Kabel oder Glasfaserkabel zur Beeinflussung auf ein biologisches System, insbesondere auf einen menschlichen oder Tierkörper, geleitet werden.

Durch die vorteilhafte Ausbildung der Vorrichtung zur Verstärkung elektromagnetischer Schwingungen zur Beeinflussung eines biologischen Systems wird es möglich, Tonsignale, die beispielsweise aus einem Tonspeicher oder einem CD-Player abgerufen werden können, zur Heilbehandlung einzusetzen. Die Schwingungen werden hierzu in vorteilhafter Weise an einer bestimmten Stelle des Körpers, insbesondere an der Stelle des Immunpunktes, eingeleitet und wirken so auf den menschlichen Körper ein. Je nach Menschentyp können hier geeignete, besonders modulierte Schwingungen zur Verfügung gestellt werden. Die Tonsignale werden also nicht wie bisher akustisch wahrgenommen. Die Tonsignale dienen zur Steuerung eines dem Lasergerät zugeschalteten Geräts, so daß die Betriebsspannung des Lasers unterschiedlich stark schwankt, beispielsweise zwischen 1 mV und 6 mV. Dadurch wird impulsartig der Laserstrahl dem menschlichen Körper zugeführt, und zwar in Abhängigkeit des entsprechend modulierten Tonsignals. Es hat sich herausgestellt, daß hierzu leichte, ruhige Musik besonders geeignet ist. Je nach Menschentyp sollte eine entsprechende Musik ausgewählt werden.

Entgegen der bisherigen Anwendung der Lasertechnik in der technischen oder thermischen Wirkung, beispielsweise beim Zertrümmern, Schneiden, Schweißen, Verdampfen, können mittels der erfindungsgemäßen Vorrichtung und durch die Wechselwirkung zwischen den Beimischungen und den elektromagnetischen Strahlen im sichtbaren Bereich spezifische Wirksamkeiten zur Entfaltung gebracht verden, und dadurch können diese Beimischungen im heilenden Sinne wirksam genutzt werden. Durch die Wechselwirkung zwischen den Lichtstrahlen und den in den Festkörpern oder Mischungen enthaltenen Substanzen wird eine wirksame Heilung in kurzer Zeit auf optimale Weise bewirkt. Mittels des Laserstrahls wird dem biologischen System die notwendige Energie in Verbindung mit den substanzspezifischen Informationen unter Ausnutzung des Ramann-Effekts zugeführt. Im vorliegenden Fall wurde beispielsweise eine Wunde zuerst im medizinisch klassischen Sinne versorgt und mit Antibiotika-Anlagen behandelt. Die Wunde hatte zu Beginn der Behandlung eine Länge von 18 cm und in der Mitte eine Breite von 2,5 cm. Die Wunde zeigte rohes Fleisch. Durch die Laserbestrahlung in Verbindung mit der in den Laserstrahl eingebrachten Substanz in einer Konzentration von 100 Vol.-% bis 10 x 10⁻²⁴ Vol.-% konnte eine optimale Heilung in kürzester Zeit, d. h. in drei Wochen, herbeigeführt werden. Dies wird in vorteilhafter Weise auch dadurch erreicht, daß der über die Substanz geführte Laserstrahl über das austretende Licht eine Information mitbekommt, die optisch dem Fingerabdruck der eingebrachten Substanz entspricht. Der austretende Laserstrahl wird in jedem Fall über den als optische Einrichtung wirkenden Festkörper bzw. die flüssige oder gasförmige Lösung mit der Beimischung geführt und auf die Wunde eingestrahlt. Die Behandlungszeit bei einer Wunde von 25 cm² kann 30 Minuten betragen. Eine derartige Behandlung über eine Woche, täglich fortgeführt, ergibt eine starke Heilreaktion, wobei die Wundgröße schließlich auf 4 cm² zusammenschrumpft und nach ca. zwei Wochen eine kreisrunde Borke aufweist. Wird ein derartiger Behandlungsprozeß über eine Zeit von drei Wochen geführt, so kann eine Wunde von ca. 23 cm² vollständig abheilen.

Dabei ist es besonders vorteilhaft, daß die Vorrichtung zur Verstärkung elektromagnetischer Schwingungen durch induzierte Emission mittels eines Festkörpers, mit Neodym dotierter Kristalle oder Gläser, Halbleiter-Dioden oder Flüssigkeiten oder Gase einsetzbar ist, wobei die aus der Vorrichtung austretenden elektromagnetischen Schwingungen über ein Kabel oder Glasfaserkabel zur Beeinflussung auf ein biologisches System, insbesondere auf einen menschlichen oder Tierkörper, geleitet werden, wobei das Kabel eine Auslaßöffnung aufweist und die aus dem Kabel austretenden Schwingungen oder Strahlen über einen Festkörper, ein Gemisch oder eine flüssige oder gasförmige Lösung zur Beeinflussung auf das biologische System geleitet werden, wobei der Festkörper, das Gemisch bzw. die flüssigen oder gasförmigen Lösungen mineralische, pflanzliche, tierische oder menschliche Extrakte bzw. Produkte oder Giftstoffe als Beimischungen enthalten.

Vorteilhaft ist es auch, daß das Kabel eine Ein- und eine Auslaßöffnung aufweist, wobei die Auslaßöffnung in die Einlaßöffnung der Behandlungssonde einmündet und zwischen den Ein- und Auslaßöffnungen des Festkörper, das Gemisch oder die gasförmige oder flüssige Lösung vorgesehen bzw. einbringbar ist.

Durch den Einsatz bestimmter Medien, die in den Lichtstrahl des Laserstrahls eingeführt werden, kann die Heilbehandlung wesentlich verbessert werden.

In weiterer Ausgestaltung der Erfindung ist es vorteilhaft, daß der Wandler zwischen einem Tonwiedergabegerät oder CD-Wiedergabegerät und der Laservorrichtung geschaltet ist.

Vorteilhaft ist es ferner, daß der Laservorrichtung ein Steuergerät zugeordnet ist, das zwischen dem Wandler und der Laservorrichtung geschaltet und/oder mit diesen wirkungsmäßig verbunden ist.

In weiterer Ausgestaltung der Erfindung ist es vorteilhaft, daß der Wandler mindestens einen Eingang für ein moduliertes Signal oder Tonsignal aufweist, das über mindestens einen Spannungsregler und mindestens eine Rückkopplung an einen Ausgang geleitet wird, der mit dem Eingang des Steuergeräts und/oder dem Eingang der Laservorrichtung verbunden ist.

Nach einem anderen Merkmal der erfindungsgemäßen Vorrichtung ist es vorteilhaft, daß das Steuergerät einen Verstärker aufweist und daß der Ausgang des Steuergeräts mit der Laservorrichtung verbunden ist.

Ferner ist es vorteilhaft, daß der Laser als Argon-Ion-Krypton-Mischgas-Laser ausgelegt ist.

Eine zusätzliche Möglichkeit ist gemäß einer Weiterbildung der erfindungsgemäßen Vorrichtung, daß der Laser als He-Ne-Laser ausgelegt ist.

In weiterer Ausgestaltung der Erfindung ist es vorteilhaft, daß der Laser als He-Cd-Laser ausgebildet ist.

Ferner ist es vorteilhaft, daß der Laser als Multiline-Singleline-, Multimod- oder als durchstimmbarer Laser ausgebildet ist.

In weiterer Ausgestaltung der Erfindung ist es vorteilhaft, daß der Laser als Dioden-Laser für Frequenzen von 5 x 10¹⁴ Hz bis 10¹⁵ Hz ausgelegt ist.

Eine zusätzliche Möglichkeit gemäß einer Weiterbildung der erfindungsgemäßen Vorrichtung besteht darin, daß der Laser als Kristall-Laser bzw. als Neodym-Yak-Laser ausgelegt ist.

Von Vorteil ist es ferner, daß in die Aufnahmevorrichtung zur Behandlung von Prellungen ein Festkörper oder gasförmiges oder flüssiges Gemisch einbringbar ist, das in der Konzentration von 10 x 10⁻³ bis 10 x 10⁻⁹ Vol.-% vorliegt.

Eine wesentliche vorteilhafte Ausführungsform erreicht man dadurch, daß in die Aufnahmevorrichtung zur Behandlung von Knochenbrüchen ein Festkörper oder gasförmiges oder flüssiges Gemisch einbringbar ist, das in der Konzentration von 10 x 10⁻³ bis 10 x 10⁻¹² Vol.-% vorliegt, wobei hierzu die Argon-Ion-Laservorrichtung eingesetzt wird.

Vorteilhaft ist es außerdem, daß in die Aufnahmevorrichtung zur Behandlung von Tumoren ein Festkörper oder gasförmiges oder flüssiges Gemisch einbringbar ist, das in der Konzentration von 10 x 10⁻⁶ Vol.-% bis 10 x 10⁻⁸ Vol.-% vorliegt, wobei hierzu die Argon-Krypton-Mischgas-Laservorrichtung eingesetzt wird.

Ferner ist es vorteilhaft, daß in die Aufnahmevorrichtung zur Behandlung von Gehirnerschütterungen ein Festkörper oder gasförmiges oder flüssiges Gemisch einbringbar ist, das in der Konzentration von 10 x 10⁻³ bis 10 x 10⁻⁹ Vol.-% vorliegt, wobei hierzu die Argon-Ion-Laservorrichtung eingesetzt wird.

Eine zusätzliche Möglichkeit ist, daß in die Aufnahmevorrichtung zur Behandlung von Stoffwechselerkrankungen ein Festkörper oder gasförmiges oder flüssiges Gemisch einbringbar ist, das in der Konzentration von 10 x 10⁻¹ bis 10 x 10⁻²⁴ Vol.-% vorliegt, wobei hierzu die Argon-Ion- und/oder Argon-Krypton-Mischgas- und/oder Dioden- und/oder Neodym-Yak- und/oder He-Ne- und/oder He-Cd-Laservorrichtung eingesetzt wird.

Eine Anordnung zur Durchführung des erfindungsgemäßen Verfahrens ist, daß in die Aufnahmevorrichtung zur Behandlung von Zellkulturen ein Festkörper oder gasförmiges oder flüssiges Gemisch einbringbar ist, das in der Konzentration von 100 Vol.-% bis 10 x 10⁻²⁴ Vol.-% vorliegt, wobei hierzu die Argon-Ion-, Argon-Krypton-, Krypton-, Dioden-, Neodym-Yak-, He-Ne-, He-Cd-Laservorrichtung eingesetzt wird.

Vorteilhaft ist es ferner, daß das Gerät oder der CD-Player, über das eine Toninformation oder Musikinformation hergestellt, brauchbar gemacht und/oder an eine andere Einrichtung weitergegeben wird, einen oder mehrere Ausgänge aufweist, wobei der eine Ausgang mit der Laservorrichtung und der andere Ausgang mit einem Kopfhörer und/oder mindestens einem Lautsprecher mittel- oder unmittelbar in Verbindung steht.

Die in dieser Beschreibung angegebenen Konzentrationen sind homogene Mischungen aus verschiedenen Stoffen. Für praktische Zwecke wird die Konzentration einer betrachteten Substanz bevorzugt in Gewichts- oder Volumenprozenten (Gewichts- oder Volumenanteil x 100 Vol.-%) angegeben. Für theoretische Überlegungen stellen der Molenbruch (Mole je Summe der Mole), die Molarität (Mole je Liter Lösung) oder die Molalität (Mole je kg Lösungsmittel) ein geeignetes Konzentrationsmaß dar.

**Weitere Vorteile und Einzelheiten der Erfindung sind in den Patentansprüchen** und in der Beschreibung erläutert und in den Figuren dargestellt.

Es zeigt:
- Figur 1: eine schematische Darstellung einer Laservorrichtung mit einem an der Auslaßöffnung der Laservorrichtung vorgesehenen Kabel bzw. Glasfaserkabel,
- Figur 2: eine Behandlungssonde bzw. eine Aufnahmevorrichtung, die über ein Kabel bzw. Glasfaserkabel mit der Laservorrichtung verbindbar ist,
- Figur 3: ein weiteres Ausführungsbeispiel einer Laservorrichtung mit einem der Laservorrichtung vorgeschalteten Tonwiedergabe- bzw. Tonspeichergerät,
- Figur 4: einen Wandler gemäß Figur 3, der vor die Laservorrichtung geschaltet ist.

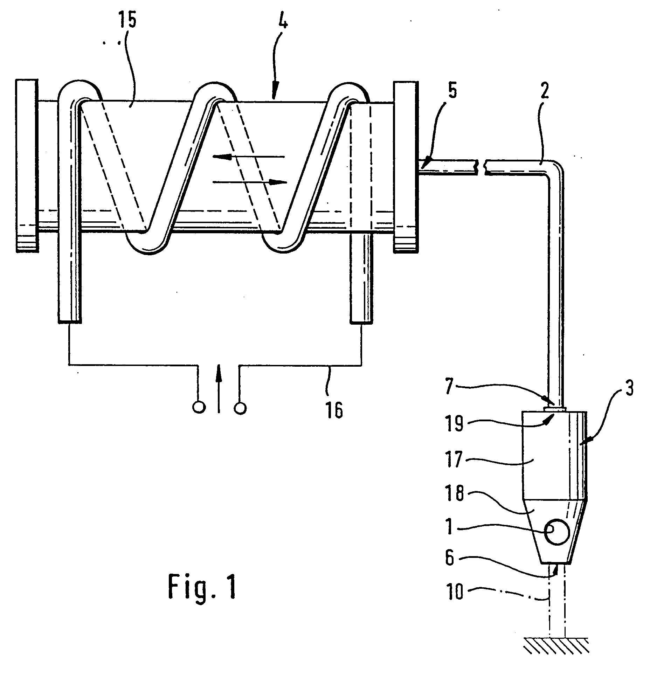

In der Zeichnung ist in Figur 1 eine Vorrichtung zur kohärenten Verstärkung elektromagnetischer Schwingungen mit 4 bezeichnet. Die Vorrichtung 4 besteht aus einem Laserrohr 15, in dem z. B. für Brandwunden ein Argon-Ion-Gas eingebracht ist. Über eine elektrische Leitung 16 wird der Laservorrichtung 4 Energie zugeführt. Die aus der Laservorrichtung 4 austretenden elektromagnetischen Schwingungen, Wellen bzw. der Laserstrahl können Frequenzen von 5 x 10¹⁴ Hz bis 10¹⁵ Hz (sichtbarer Bereich) aufweisen. Je nach Heilbehandlung bzw. Anwendungsart kann die Laservorrichtung so ausgelegt sein, daß sie im Ultraviolett- oder Infrarotbereich eingesetzt werden kann. Die hier angegebenen Frequenzen geben den sichtbaren Anteil (Licht) des elektromagnetischen Spektrums wieder.

Ein Laserstrahl bzw. ein Lichtstrahl 10 wird über ein Kabel 2 geleitet, dessen Einlaßöffnung 5 mit der Laservorrichtung 4 und dessen Auslaßöffnung und mit einer Einlaßöffnung 19 einer Behandlungssonde bzw. Aufnahmevorrichtung 3 in Verbindung steht. Über die Auslaßöffnung 6 trifft der Laserstrahl 10 nach außen und wird auf ein biologisches System 11 gerichtet.

Die Aufnahmevorrichtung 3 besteht aus einem zylinderförmigen Gehäuseteil 17 mit einem sich daran anschließenden, sich nach vorne verjüngenden Gehäuseteil 18. Über die gesamte Länge der Aufnahmevorrichtung 3 erstreckt sich eine Längsöffnung 9, die in die Auslaßöffnung 6 mündet. Über die Längsöffnung 9 wird also der Licht- bzw. Laserstrahl 10 geleitet, der dann über die in der Aufnahmevorrichtung 3 vorgesehene Auslaßöffnung 6 geführt wird, auf das biologische System 11 bzw. einen menschlichen Körper, einen Tierkörper oder einen Pflanzlichen Körper auftrifft und dort die gewünschte Heilwirkung bzw. Veränderung im biologischen System herbeiführt. Im Bereich der Auslaßöffnung 6 der Aufnahmevorrichtung 3 befindet sich eine Queröffnung 1, die die Längsöffnung 9 in einem Winkel, in vorteilhafter Weise in einem rechten Winkel, schneidet.

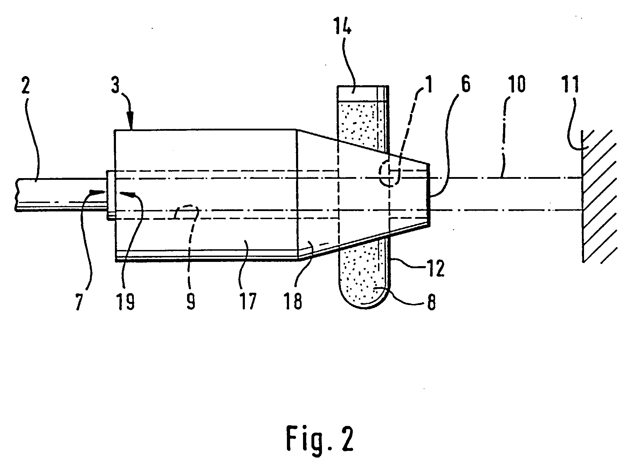

Wie aus Figur 2 hervorgeht, ist in die Queröffnung 1 ein Behälter 12 derart eingeführt, daß er den Strahlenkranz des Laserstrahls in einem Winkel von ca. 90° schneidet. Dadurch werden die Laserstrahlen über bzw. durch den Behälter 12 geleitet. Der Behälter 12 ist deshalb in vorteilhafter Weise aus Glas, insbesondere aus Quarzglas, gebildet, so daß fast keine Verluste auftreten, wenn der Laserstrahl das Quarzglas passiert.

Der Behälter 12 kann in vorteilhafter Weise zylinderförmig ausgebildet sein. Es ist jedoch auch möglich, den Behälter 12 konisch auszubilden und die entsprechende Queröffnung 1 ebenfalls der konischen Ausbildung des Behälters 12 anzupassen, so daß eine Klemmverbindung zwischen der Aufnahmevorrichtung 3 und dem Behälter 12 erreicht wird.

Der Behälter 12 dient zur Aufnahme eines oder mehrerer Festkörper bzw. zur Aufnahme von Substanzen in pulvriger Form oder zur Aufnahme einer gasförmigen oder flüssigen Lösung. In den Behäter 12 kann zusätzlich eine Trägerlösung, z. B. Wasser und Äthanol im Verhältnis 1:1, eingefüllt werden. In die Trägerlösung werden die Beimischungen oder Substanzen in einer Konzentration von 50 Vol.-% bis zu 10 x 10⁻²⁴ Vol.-% in abnehmender Tendenz zugegeben. In diese Trägerlösung werden z. B. mineralische, pflanzliche, tierische oder menschliche Extrakte bzw. Produkte oder Giftstoffe zusätzlich eingegeben. Diese Zusatzstoffe bezeichnet man als Beimischung 8, die entscheidend für die Positive Beeinflussung des biologischen Systems 11 oder die entsprechende medizinische Behandlung ist.

Die Befüllung des Behälters 12 kann also eine echte Lösung, ein Lösungsgemisch oder eine kolloidale Lösung sein. Je nach Zusammensetzung der Beimischung kann auch auf die Trägerlösung verzichtet werden.

Der Laserstrahl 10 wird über den Behälter 12 mit den Substanzen bzw. Beimischungen geleitet und trifft dann auf das biologische System 11. In jeder Atom- oder Molekülverbindung wird in vorteilhafter Weise die Lichtstrahlung spezifisch gestreut (Ausnutzung des Ramann-Effekts), und das austretende Licht enthält praktisch den Fingerabdruck der Beimischungen. Wird beispielsweise ein Argon-Ionen-Laser eingesetzt, liefert dieser Laserstrahl die entsprechende Grundenergie für die biologischen Prozesse. Der austretende Laserstrahl gibt dann die Informationen an das biologische System weiter und bewirkt den Heilungsprozeß z. B. bei einer Brandwunde.

Der Abstand zwischen dem Austrittsende der Aufnahmevorrichtung 3 und dem biologischen System 11 ist beim Behandlungsprozeß meist konstant. Vergrößert man den Abstand zwischen dem Austrittsende der Aufnahmevorrichtung 3 und dem biologischen System 11, so wird auch eine größere Fläche des biologischen Systems mittels des Laserstrahls 10 erreicht.

Wird beispielsweise bei der Wundbehandlung oder auch einer anderen Behandlungsart der Neodym-Yak-Laser eingesetzt, so ist es vorteilhaft, wenn die Wellenlänge für 532 nm (grün) und 473 nm (blau) verwendet wird.

Besonders vorteilhaft ist es, wenn die Laseremission im sichtbaren Bereich, also zwischen 650 nm und 300 nm (UV), liegt. Die Wahl der Wellenlänge hängt davon ab, welches biologische System 11 bestrahlt werden soll. Infrarotes Licht sollte nicht für menschliche Zellen verwendet werden, da es menschliche Zellen abtöten kann.

Wird beispielsweise die Laservorrichtung zur Tumorbehandlung eingesetzt, so besteht der Laser aus einem Argon-Ion-Krypton-Ion-Mischgaslaser zwischen 2 mm und 100 mm.

In diesem Fall werden folgende Beimischungen in den Behälter eingegeben:
a) Lokal: Extrakte aus Krankheitserregern von Tuberkulose und/oder Gonorrhoe und/oder Syphilis in Konzentration von 10 x 10⁻⁶ Vol.-% bis 10 x 10⁻⁸ Vol.-%.
b) Limbisches System (Gehirn): Auszug aus Salamandra 10 x 10⁻⁶ Vol.-% bis 10 x 10⁻⁸ Vol.-%.

Bei der Behandlung von Knochenbrüchen setzt man in vorteilhafter Weise einen Argon-Ion-Laser zwischen 2 mm und 100 mm ein.

Bei dieser Behandlung setzt man Substanzen als Beimischung ein, wie sie auf Seite 15 aufgelistet sind.

Aus vorstehenden Ausführungen ergibt sich, daß zur medizinischen Behandlung des biologischen Systems 11 und je nach Krankheitsart ein ganz bestimmter Lasertyp mit einer entsprechenden Sonde eingesetzt werden muß, wobei jeder Sonde bzw. Aufnahmevorrichtung eine bestimmte Beimischung 8 eingebracht wird.

Bei einem 1927 geborenen Patienten wurde eine offene, nichtheilende Wunde am linken Oberschenkel festgestellt. Diese Wunde ließ sich mit keiner klassischen Versorgung (z. B. Antibiotikagaben, chirurgische Wundrandbehandlung) positiv beeinflussen. Die Behandlung dieser Wunde erfolgte mit dem beschriebenen Argon-Ion-Laser, wobei die emittierten Frequenzen im sichtbaren Bereich lagen. Die Leistungsaufnahme der Laservorrichtung 4 lag bei 50 mW. Der Laserstrahl 10 wurde über die im Quarzglasbehälter 12 aufgenommenen Substanzen 8 auf die Brandwunde 11 geleitet. In der Trägerlösung waren mehrere Substanzen in folgender Konzentration enthalten: Auszug aus Aconitum 10 x 10⁻⁴ Vol.-% und Auszug aus Arnika 10 x 10⁻³ Vol.-%.

Der austretende Laserstrahl 10 wurde, wie bereits erwähnt, über den als optische Einrichtung wirkenden Behälter 12 auf die gesamte Wunde gestrahlt. Die Behandlungszeit betrug jeweils 30 Minuten. Schon nach einer Woche trat eine starke Heilreaktion ein. Die Wunde, die bei Beginn der Laserbestrahlung 10 cm lang und in der Mitte 2,5 cm breit, d. h. 25 cm² groß war, hatte nach einer Woche Behandlung eine Größe von 2 cm Durchmesser und nach zwei Wochen eine Größe von 7 mm Durchmesser. Nach drei Wochen war die Wunde vollständig abgeheilt.

Die Laserbestrahlung wurde erfolgreich bei Wundbehandlung, Knochenbrüchen, Zerrungen, Stauchungen und Gehirnerschütterungen eingesetzt.

Bei einer schweren Gehirnerschütterung und einer nach fünf Minuten aufgenommenen Laserbestrahlung konnte schon nach zehnminütiger Behandlung völlige Beschwerdefreiheit und normale neurologische Reaktion festgestellt werden.

Je nach Behandlungsart wird der entsprechende Laser mit dem zugehörigen, eine entsprechende Beimischung aufweisenden Behälter 12 eingesetzt.

Mit der Laserbehandlung bzw. Bestrahlung kann auch der Stoffwechsel positiv beeinflußt werden. Die entsprechenden Beimischungen enthalten Auszüge aus Pflanzenextrakten, aus tierischen und aus menschlichen Produkten, ferner Auszüge aus Viren, Bakterien, Pilzen und anderen Krankheitserregern sowie Auszüge aus Tumoren und pathologischen Sekreten.

Bei Organspenden können Zellkulturen und lebende Zellen positiv beeinflußt werden. Ferner ist es vorteilhaft, die Laserbestrahlung bei Mikroorganismen und auch Viren außerhalb von Lebewesen einzusetzen.

Nachstehende Lösungen wurden für die Wundbehandlung in folgender Konzentration erfolgreich eingesetzt:
- Auszug aus Aconitum: 10 x 10⁻⁴ Vol.-%
- Auszug aus Arnika: 10 x 10⁻³ Vol.-%
- Auszug aus Belladonna: 10 x 10⁻⁴ Vol.-%
- Auszug aus Bellis: 10 x 10⁻⁴ Vol.-%
- Auszug aus Calendula: 10 x 10⁻⁵ Vol.-%
- Auszug aus Chamomilla: 10 x 10⁻⁴ Vol.-%
- Auszug aus Echinacea ang.: 10 x 10⁻⁴ Vol.-%
- Auszug aus Echinacea purp.: 10 x 10⁻⁴ Vol.-%
- Auszug aus Hamamelis: 10 x 10⁻³ Vol.-%
- Auszug aus Millefolium: 10 x 10⁻⁴ Vol.-%
- Auszug aus Symphytum: 10 x 10⁻⁹ Vol.-%
- Lösung Hepar sulf.: 10 x 10⁻⁹ Vol.-%
- Lösung Merkurius sol.: 10 x 10⁻⁹ Vol.-%
- Lösung Quarz: 10 x 10⁻⁶ Vol.-%

Diese und andere Konzentrationen werden in den Behälter 12 eingegeben. Der Behälter 12 funktioniert als optischer Körper bzw. als Linse und weitet den Laserstrahl 10 auf. Beim Auftreffen des Laserstrahls auf ein Atom oder ein Molekül wird der Laserstrahl gestreut, d. h. es wird der erwähnte Ramann-Effekt genutzt. Der austretende Laserstrahl enthält den erwähnten optischen Fingerabdruck der vorstehenden Substanzen und nimmt Einfluß auf die Behandlung des biologischen Systems 11. Die Streustrahlen produzieren ein Biophotonenfeld.

Für die Behandlung von Knochenbrüchen werden Argon-Ion-Laser eingesetzt und Substanzen 8 in den Behälter 12 eingebracht. Diese Beimischung oder Substanz weist eine Konzentration von 10 x 10⁻³ bis 10 x 10⁻¹² Vol.-% auf.

Für die Behandlung der Wildrosen wird ein roter Diodenlaser 632 nm eingesetzt und ein Gemisch-Quarz in der Konzentration von 10 x 10⁻⁶ eingegeben.

Wird beispielsweise die Laservorrichtung zur Allergiebehandlung eingesetzt, so besteht der Laser aus einem roten He-Ne- oder Diodenlaser bei 632 nm und einem grünen He-Ne/Neodym-Yak-Laser.

In diesem Fall werden folgende Beimischungen in den Behälter gegeben: Auszug aus Dioscorea vilosa in der Konzentration von 10 x 10⁻⁵ Vol.-% und Kalium carb. in der Konzentration von 10 x 10⁻⁶ Vol.-%.

Zur Behandlung von Haarausfall besteht der Laser aus einem grünen He-Ne- oder Neodym-Yak-Laser.

In diesem Fall werden folgende Beimischungen in den Behälter gegeben: Extrakt aus Tuberkulose und/oder Placenta und/oder Extrakt aus Pel talpae in der Konzentration von 10 x 10⁻³ Vol.-% bis 10 x 10⁻⁹ Vol.-%.

Wird die Laservorrichtung zur Behandlung von Darmentzündung eingesetzt, so besteht der Laser aus einem grünen He-Ne- oder Neodym-Yak-Laser im Wechsel mit einem gelben He-Ne-Laser.

In diesem Fall werden folgende Beimischungen in den Behälter gegeben: Extrakte aus Krankheitserregern in der Konzentration von 10 x 10⁻⁴ bis 10 x 10⁻¹² Vol.-%.

Wird beispielsweise die Laservorrichtung zur Behandlung von Durchblutungsstörungen eingesetzt, so besteht der Laser aus einem roten He-Ne-Diodenlaser.

In diesem Fall werden folgende Beimischungen in den Behälter gegeben: Extrakte aus Krankheitserregern und/oder Schlangengiften in der Konzentration von 10 x 10⁻⁴ Vol.-% bis 10 x 10⁻¹² Vol.-%.

Zur Behandlung von Ekzemen besteht der Laser aus einem grünen Laser im Wechsel mit einem Argon-Ion-Multiline-Laser.

In diesem Fall werden folgende Beimischungen in den Behälter gegeben: Extrakte aus Krankheitserregern in der Konzentration von 10 x 10⁻⁴ bis 10 x 10⁻¹² Vol.-%.

Für die Behandlung von Abwehrschwäche (Immunschwäche) besteht der Laser aus einem roten He-Ne-Diodenlaser.

In diesem Fall werden folgende Beimischungen in den Behälter gegeben: Extrakte aus Krankheitserregern in der Konzentration von 10 x 10⁻⁴ bis 10 x 10⁻¹² Vol.-%.

Zur Behandlung einer Gallenkolik besteht der Laser aus einem grünen He-Ne- oder Neodym-Yak-Laser.

In diesem Fall werden folgende Beimischungen in den Behälter gegeben: Extrakte aus Krankheitserregern, Magnesium phosphat und Calcium phosphat in der Konzentration von 10 x 10⁻⁴ Vol.-% bis 10 x 10⁻¹² Vol.-%.

Wird beispielsweise die Laservorrichtung zur Behandlung von Magenbeschwerden eingesetzt, so besteht der Laser aus einem roten He-Ne-Diodenlaser.

In diesem Fall werden folgende Beimischungen in den Behälter gegeben: Extrakte aus Krankheitserregern in der Konzentration von 10 x 10⁻⁴ bis 10 x 10⁻¹² Vol.-%.

Wird die Laservorrichtung zur Behandlung von Gelenkbeschwerden eingesetzt, so besteht der Laser aus einem Argon-Ion-Multiline-Laser.

In diesem Fall werden folgende Beimischungen in den Behälter gegeben: Extrakte aus Krankheitserregern in der Konzentration von 10 x 10⁻⁴ bis 10 x 10⁻¹² Vol.-%.

Zur Behandlung von Verdauungsorganen und der Nieren besteht der Laser aus einem gelben und einem grünen He-Ne-Laser.

In diesem Fall werden folgende Beimischungen in den Behälter gegeben: Extrakte aus Krankheitserregern in der Konzentration von 10 x 10⁻⁴ bis 10 x 10⁻¹² Vol.-% und/oder Mineralien in der Konzentration von 10 x 10⁻³ bis 10 x 10⁻²⁴ Vol.-% und/oder Extrakt aus Salamandra in der Konzentration von 10 x 10⁻⁸ Vol.-%.

Wird die Laservorrichtung zur Behandlung von Herpes und Herpes zoster eingesetzt, so besteht der Laser aus einem Argon-Ion-Multiline-Laser.

In diesem Fall werden folgende Beimischungen in den Behälter gegeben: Extrakt aus Tuberkulose in der Konzentration von 10 x 10⁻⁶ Vol.-%.

Für die Behandlung von Nervenentzündungen (Ischialgie) besteht der Laser aus einem Argon-Ion-Multiline-Laser.

In diesem Fall werden folgende Beimischungen in den Behälter gegeben: Extrakt aus Tuberkulose in der Konzentration von 10 x 10⁻⁶ Vol.-%.

Zur Behandlung von Herzschwäche, pektanginöse Beschwerden, besteht der Laser aus einem orangefarbenen He-Ne-Laser.

In diesem Fall werden folgende Beimischungen in den Behälter gegeben: Extrakte aus Krankheitserregern in der Konzentration von 10 x 10⁻⁴ bis 10 x 10⁻¹² Vol.-% und/oder Mineralien in der Konzentration von 10 x 10⁻³ bis 10 x 10⁻²⁴ Vol.-% und/oder Extrakt aus Salamandra in der Konzentration von 10 x 10⁻⁸ Vol.-%.

Wird beispielsweise die Laservorrichtung zur Behandlung von Migräne eingesetzt, so besteht der Laser aus einem Argon-Ion-Multiline-Laser und einem roten He-Ne-Dioden-Laser.

In diesem Fall werden folgende Beimischungen in den Behälter gegeben: Extrakt aus Tuberkulose in der Konzentration von 10 x 10⁻⁶ Vol.-%.

Zur Behandlung von Neurodermitis besteht der Laser aus einem Argon-Krypton-Mischgaslaser und einem roten He-Ne-Dioden-Laser.

In diesem Fall werden folgende Beimischungen in den Behälter gegeben: Extrakte aus Krankheitserregern in der Konzentration von 10 x 10⁻⁴ bis 10 x 10⁻¹² Vol.-% und/oder Mineralien in der Konzentration von 10 x 10⁻³ bis 10 x 10⁻²⁴ Vol.-% und/oder Extrakt aus Salamandra in der Konzentration von 10 x 10⁻⁸ Vol.-% und einem Auszug aus Dioscorea vilosa in der Konzentration von 10 x 10⁻⁵ Vol.-% und Kalium carb. in der Konzentration von 10 x 10⁻⁶ Vol.-%.

Wird die Laservorrichtung zur Behandlung von Parkinson eingesetzt, so besteht der Laser aus einem Argon-Ion-Multiline-Laser und einem Argon-Krypton-Mischgaslaser.

In diesem Fall werden folgende Beimischungen in den Behälter gegeben: Extrakte aus Krankheitserregern in der Konzentration von 10 x 10⁻⁴ bis 10 x 10⁻¹² Vol.-% und Extrakte aus Hormonen in der Konzentration von 10 x 10⁻⁶ bis 10 x 10⁻²⁴ Vol.-%.

Für die Behandlung von Drüsenstörungen besteht der Laser aus einem Argon-Ion-Multiline-Laser und einem Argon-Krypton-Mischgaslaser und einem grünen He-Ne- oder Neodym-Yak-Laser.

In diesem Fall werden folgende Beimischungen in den Behälter gegeben: Extrakte aus Krankheitserregern in der Konzentration von 10 x 10⁻⁴ bis 10 x 10⁻¹² Vol.-% und Extrakte aus Hormonen in der Konzentration von 10 x 10⁻⁶ bis 10 x 10⁻²⁴ Vol.-%.

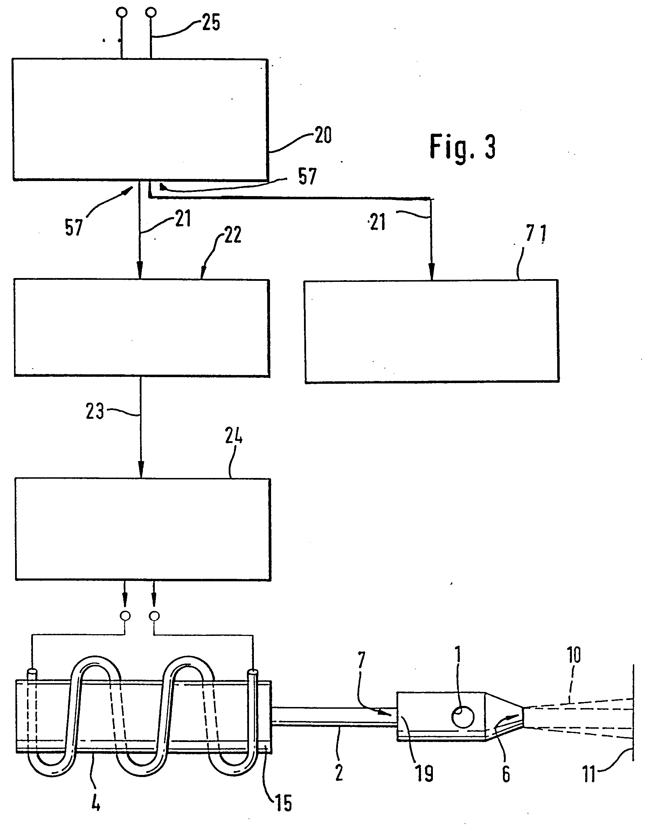

In Figur 3 ist ein weiteres Ausführungsbeispiel der Vorrichtung 4 zur Verstärkung elektromagnetischer Schwingungen zur Beeinflussung eines biologischen Systems 11 dargestellt. Zu dieser Vorrichtung gehört ein Aufnahme- oder Wiedergabegerät für ein analoges oder digitales Tonsignal und/oder ein Speichergerät 20 mit einer Eingangsleitung 25, die an eine Stromquelle anschließbar ist. Ein Speichergerät, das modulierte Signale, insbesondere Tonsignale, aufnehmen kann, wird nachstehend beschrieben. Es wird vor das Lasergerät geschaltet und beeinflußt dadurch die Stromversorgung in Abhängigkeit der Intensität des Tonsignals.

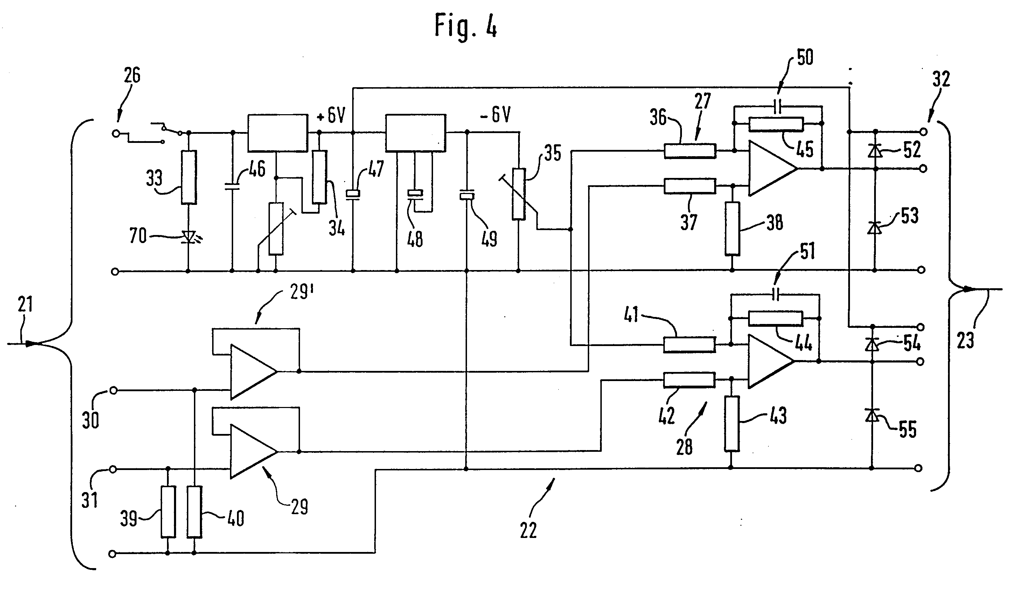

Wie aus Figur 3 hervorgeht, ist der CD-Player bzw. das Tonwiedergabegerät 20 über eine Leitung 21 mittel- oder unmittelbar mit einem Eingang eines Wandlers 22 verbunden. Der Wandler 22 ist in Figur 4 im einzelnen dargestellt.

Der Ausgang des Wandlers 22 steht über eine Leitung 23 mit einem Steuergerät 24 für den Laser in Verbindung. Die Ausgänge des Steuergeräts 24 sind mit den Eingängen der Stromversorgung der Laservorrichtung 4 verbunden.

Wird beispielsweise das Tonsignal aus dem CD-Player über die Leitung 21 der Steuervorrichtung für den Laser zugeführt, so wird in Abhängigkeit der Tonsignale, die über den Wandler in Stromsignale umgewandelt werden, der Laser mit einer unterschiedlichen Spannung versorgt, so daß sich die Intensität des Laserstrahls 4 in Abhängigkeit des Tonsignals aus dem CD-Player 20 kontinuierlich ändert. Dieser sogenannte modulierte oder beeinflußbare Laserstrahl 4 wird dann über die Behandlungssonde 3 auf das biologische System bzw. den menschlichen Körper 11 geleitet. Es ist hierzu vorteilhaft, daß der Laserstrahl 4 am Immunpunkt in den menschlichen Körper eindringt. Die in den Behälter 12 eingeführten Substanzen werden, wie eingangs beschrieben, dem Krankheitsbild entsprechend variiert.

Die Laservorrichtung 4 kann beispielsweise als Dioden-Laser ausgebildet sein und hierzu drei Eingänge aufweisen. Der eine Eingang dient zur Versorgungsspanne, die in vorteilhafter Weise zwischen 3 und 10 Volt, insbesondere aber zwischen 4 und 5 Volt liegt. Weiter weist der Laser einen Eingang für ein positives Modulationssignal und einen weiteren Eingang für ein negatives Modulationssignal auf.

Der Modulationseingang wird über sehr geringe Steuerströme (< 500 mA) geschaltet. Er ist, wie bereits erwähnt, für Spannungen zwischen 2 und 10 Volt, insbesondere zwischen 6 und 8 Volt, ausgelegt.

Das aus dem CD-Player kommende und dann umgewandelte Signal wird über den Wandler 22 an den Eingang (Versorgungsspannung) der Laservorrichtung 4 geleitet. Durch die unterschiedlichen Frequenzen sind auch die Ströme, die aus dem Wandler austreten, unterschiedlich stark und liegen zwischen 6 und 8 Volt. Das modulierte Stromsignal beeinflußt die Intensität des austretenden Laserstrahls an der Öffnung 6 der Vorrichtung 4. Auf diese Weise treffen unterschiedlich starke Laserstrahlen auf den menschlichen Körper 11 und beeinflussen dadurch den Heilvorgang. Obwohl es möglich ist, daß der Laserstrahl, der aus der Laservorrichtung 4 austritt, direkt auf den menschlichen Körper 11 geleitet wird, ist es besonders vorteilhaft, wenn der Laserstrahl über eine in den Behälter 12 aufgenommene Substanz 8 geführt wird. Hierdurch wird die Heilwirkung wesentlich verbessert. Die im Behälter 12 aufgenommenen Substanzen 8 hängen, wie bereits erwähnt, von der Behandlungsart ab.

In Figur 4 ist der Wandler bzw. der Informationswertgeber 22 dargestellt. Der Wandler 22 dient zur Umwandlung der elektrischen Größen, die aus dem Tonwiedergabegerät stammen. In Frage kommen Drücken, Dehnen, Tonschwingung, magnetische oder optische Strahlungsgrößen. Sie alle wandelt man in entsprechende elektrische Größen um und kann sie so ohne Störung des Vorgangs Präzise an das Steuergerät 24 bzw. direkt an den Laser weitergeben. Diese unterschiedlichen Stromstöße bzw. Ströme, die von dem Tonsignal beeinflußt werden, gehen auf das Steuergerät des Lasers über und beeinflussen, wie bereits erwähnt, den Laserstrahl. Der Wandler weist hierzu zwei Eingänge 30 und 31 (Kanal I und Kanal II) auf, der über je einen Spannungsregler 29, 29' und über eine Rückkopplungseinrichtung 51 mit dem Ausgang 23 verbunden ist. Der Wandler 22 läßt sich über einen Schalter 26 an eine Spannungsquelle anschließen. Die Stromversorgungsseite des Wandlers 22 weist Widerstände 33, 34, 35, 36, 37, 38, 45 auf, die über entsprechende Leitungen mit den Versorgungsleitungen verbunden sind. Ferner weist der Wandler die Kondensatoren 46, 47, 48, 49 und 50 sowie Dioden 52, 53 und 70 auf. In den Kanälen 1 und 2 befinden sich hinter den Eingängen 30 und 31 Widerstände 39, 40, 41, 42, 43 und 44 sowie die Dioden 54 und 55. Einzelheiten der Anschlüsse der Widerstände, Dioden und Kondensatoren, des Spannungsreglers und der Rückkopplung sowie der Funktion ergeben sich aus Figur 4.

Vorteilhaft ist es besonders, klassische Musik oder eine andere ausgewogene Musik zu verwenden. Jede andere Musik, beispielsweise Technomusik oder gepulste Laser, technisch bedingt oder willkürlich erzeugt, z. B. 1000 Hertz, was im weitesten Sinne als akustisches Signal verstanden wird, kann durch ihre Einseitigkeit schädliche Wirkungen auf die innere Balance biologischer Systeme bewirken.

Die Anwendung von Musik, insbesondere harmonische, ruhige Themen, schützt das biologische System vor Einseitigkeit.

Ein monotoner, bleibender Brummton, wie er auf einer Baustelle auftritt, kann auf die Dauer schmerzhaft sein.

Die Behandlung mittels Musik bewirkt unter Einsatz harmonischer Klangfolgen eine Wirkungssteigerung und vermeidet eine einseitige, monotone Beeinflussung des biologischen Systems.

Wie aus Figur 3 hervorgeht, ist der CD-Player bzw. das Tonwiedergabegerät 20 über eine Leitung 21 mittel- oder unmittelbar mit einem Eingang eines Wandlers 22 verbunden. Der Wandler 22 ist in Figur 4 im einzelnen dargestellt.

Das Gerät oder das Aufnahmegerät für ein analoges oder digitales Tonsignal, Tonwiedergabegerät, Speichergerät, CD-Player 20, über das eine Toninformation oder Musikinformation hergestellt, brauchbar gemacht und/oder an eine andere Einrichtung weitergegeben wird, weist einen oder mehrere Ausgänge 57, 57' auf. Der eine Ausgang 57 steht mittelbar mit der Laservorrichtung 4 und der andere Ausgang 57' mittel- oder unmittelbar mit einem Kopfhörer und/oder mindestens einem Lautsprecher 71 in Verbindung. Hierdurch wird der zu behandelnde Patient auf zweierlei Weise mit modulierten Schwingungen bzw. über Musik beeinflußt, was einen sehr positiven Einfluß auf den Krankheitsverlauf des Patienten hat.

Im Ausführungsbeispiel ist der CD-Player bzw. das Tonwiedergabegerät 20 durch jedes andere Gerät zu ersetzen, über das modulierte Töne, beispielsweise Musik, hergestellt und an ein anderes Gerät weitergegeben werden können.

Im Ausführungsbeispiel gemäß Figur 3 ist am Ausgang eines derartigen Geräts, beispielsweise an dem CD-Player 20, parallel zum Ausgang 57 ein Ausgang 57' vorgesehen, der entweder mittel- oder unmittelbar mit Lautsprechern oder mit Kopfhörern in Verbindung steht. Der Ausgang 57' kann entweder über eine elektrische Leitung mit den Kopfhörern oder den Lautsprechern oder auch über eine Funkstrecke verbunden sein. Hierdurch soll sichergestellt werden, daß die Musikimpulse, die normalerweise an die Vorrichtung 4 bzw. an den Laser geleitet werden, parallel auch an einen Kopfhörer oder an Lautsprecher weitergegeben werden können. Hierdurch wird die zu behandelnde Person einmal indirekt durch die nicht hörbaren Impulssignale aus dem Laser 4 beeinflußt bzw. behandelt und parallel dazu über hörbar gemachte akustische Signale zusätzlich behandelt. Dadurch hat sich herausgestellt, daß der Behandlungserfolg wesentlich verbessert werden kann, wenn die Tonimpulse auf zweierlei Art an die zu behandelnde Person herangebracht werden.

### Bezugszeichenliste

- 1: Queröffnung
- 2: Kabel, Glasfaserkabel
- 3: Behandlungssonde, Aufnahmevorrichtung, Sonde
- 4: Vorrichtung zur kohärenten Verstärkung elektromagnetischer Schwingungen (Laservorrichtung) = Laser
- 5: Einlaßöffnung des Kabels 2
- 6: Auslaßöffnung der Vorrichtung 4
- 7: Auslaßöffnung des Kabels 2
- 8: Beimischung; Festkörper, Gemisch, flüssige oder gasförmige (Substanz) Lösung
- 9: Längsöffnung
- 10: elektromagnetische Schwingungen, Wellen, Laserstrahl bzw. Strahleingang des Laserstrahls
- 11: biologisches System, menschlicher Körper, Tierkörper (Brandwunde)
- 12: Behälter
- 14: Verschlußeinrichtung
- 15: Laserrohr
- 16: elektrische Leitung
- 17: Gehäuseteil der Aufnahmevorrichtung 3
- 18: Gehäuseteil der Aufnahmevorrichtung 3
- 19: Einlaßöffnung der Aufnahmevorrichtung 3
- 20: Aufnahmegerät für ein analoges oder digitales Tonsignal, Tonwiedergabegerät, Speichergerät, CD-Player
- 21: Leitung
- 22: Tonsignalaufnehmer, Wandler
- 23: Leitung des Wandlers 22, Ausgang
- 24: Steuergerät
- 25: Eingangsleitung
- 26: Schalter
- 27: Rückkopplung
- 28: Rückkopplung
- 29: Spannungsregler
- 29': Spannungsregler
- 30: Eingang, Kanal I
- 31: Eingang, Kanal II
- 33: Widerstand
- 34: Widerstand
- 35: Widerstand
- 36: Widerstand
- 37: Widerstand
- 38: Widerstand
- 39: Widerstand
- 40: Widerstand
- 41: Widerstand
- 42: Widerstand
- 43: Widerstand
- 44: Widerstand
- 45: Widerstand
- 46: Kondensator
- 47: Kondensator
- 48: Kondensator
- 49: Kondensator
- 50: Kondensator
- 51: Rückkopplung
- 52: Diode
- 53: Diode
- 54: Diode
- 55: Diode
- 57: Ausgang CD-Abspielgerät
- 70: Diode
- 71: Lautsprecher

## Patentansprüche

1. Vorrichtung (4) zur Verstärkung elektromagnetischer Schwingungen zur Beeinflussung eines biologischen Systems, **gekennzeichnet durch folgende Merkmale:**
a) die Vorrichtung (4) weist ein Aufnahme- und/oder Wiedergabegerät für ein analoges oder digitales Tonsignal und/oder ein Speichergerät (25) für ein analoges oder digitales Tonsignal auf,
b) der Ausgang (57) zumindest eines dieser Geräte gibt das Tonsignal an einen mit diesem verbundenen Tonsignalaufnehmer oder Wandler (22) weiter,
c) der Tonsignalaufnehmer oder Wandler (22) ist mit einer Vorrichtung zur kohärenten Verstärkung elektromagnetischer Schwingungen bzw. einer Vorrichtung zur Verstärkung elektromagnetischer Schwingungen oder einer Laservorrichtung (4) wirkungsmäßig verbunden,
d) die aus der Laservorrichtung (4) austretenden elektromagnetischen Schwingungen werden über ein Kabel oder Glasfaserkabel (2) zur Beeinflussung auf ein biologisches System, insbesondere auf einen menschlichen oder Tierkörper (11), geleitet.

2. Vorrichtung nach Anspruch 1, **dadurch gekennzeichnet**, daß die Vorrichtung (4) zur Verstärkung elektromagnetischer Schwingungen durch induzierte Emission mittels eines Festkörpers, mit Neodym dotierter Kristalle oder Gläser, Halbleiter-Dioden oder Flüssigkeiten oder Gase einsetzbar ist, wobei die aus der Vorrichtung austretenden elektromagnetischen Schwingungen über ein Kabel oder Glasfaserkabel (2) zur Beeinflussung auf ein biologisches System, insbesondere auf einen menschlichen oder Tierkörper (11), geleitet werden, wobei das Kabel (2) eine Auslaßöffnung (7) aufweist und die aus dem Kabel (2) austretenden Schwingungen oder Strahlen über einen Festkörper, ein Gemisch oder eine flüssige oder gasförmige Lösung (8) zur Beeinflussung auf das biologische System (11) geleitet werden, wobei der Festkörper, das Gemisch bzw. die flüssigen oder die gasförmigen Lösungen mineralische, pflanzliche, tierische oder menschliche Extrakte bzw. Produkte oder Giftstoffe als Beimischungen enthalten.

3. Vorrichtung nach Anspruch 1 oder 2, **dadurch gekennzeichnet**, daß das Kabel (2) eine Einlaßöffnung (5) und die Auslaßöffnung (7) aufweist, wobei die Auslaßöffnung (7) in die Einlaßöffnung (19) der Behandlungssonde einmündet und zwischen den Ein- und Auslaßöffnungen (19, 6) der Festkörper, das Gemisch oder die gasförmige oder flüssige Lösung (8) vorgesehen bzw. einbringbar ist.

4. Vorrichtung nach Anspruch 1 oder 2 **dadurch gekennzeichnet,** daß der Wandler (22) zwischen einem Tonwiedergabegerät oder CD-Wiedergabegerät (20) und der Laservorrichtung (4) geschaltet ist.

5. Vorrichtung nach Anspruch 1, **dadurch gekennzeichnet,** daß der Laservorrichtung (4) ein Steuergerät (24) zugeordnet ist.

6. Vorrichtung nach einem der vorhergehenden Ansprüche, **dadurch gekennzeichnet**, daß das Steuergerät (24) zwischen dem Wandler (22) und der Laservorrichtung (4) geschaltet und/oder mit diesen wirkungsmäßig verbunden ist.

7. Vorrichtung nach Anspruch 1, **dadurch gekennzeichnet,** daß der Wandler (22) mindestens einen Eingang für ein moduliertes Signal oder Tonsignal aufweist.

8. Vorrichtung nach einem der vorhergehenden Ansprüche, **dadurch gekennzeichnet,** daß der Wandler (22) mindestens einen Eingang für ein moduliertes Signal oder Tonsignal aufweist, das über mindestens einen Spannungsregler (29) und mindestens eine Rückkopplung (27, 28) an einen Ausgang (23) geleitet wird, der mit dem Eingang des Steuergeräts (24) und/oder dem Eingang der Laservorrichtung (4) verbunden ist.

9. Vorrichtung nach einem der vorhergehenden Ansprüche, **dadurch gekennzeichnet**, daß das Steuergerät (24) einen Verstärker aufweist.

10. Vorrichtung nach einem der vorhergehenden Ansprüche, **dadurch gekennzeichnet,** daß der Ausgang des Steuergeräts (24) mit der Laservorrichtung (4) verbunden ist.

11. Vorrichtung nach Anspruch 10, **dadurch gekennzeichnet,** daß der Laser (4) als Argon-Ion-Krypton-Mischgas-Laser ausgelegt ist.

12. Vorrichtung nach einem der vorhergehenden Ansprüche, **dadurch gekennzeichnet,** daß der Laser (4) als He-Ne-Laser ausgelegt ist.

13. Vorrichtung nach einem der vorhergehenden Ansprüche, **dadurch gekennzeichnet,** daß der Laser (4) als He-Cd-Laser ausgebildet ist.

14. Vorrichtung nach einem der vorhergehenden Ansprüche, **dadurch gekennzeichnet,** daß der Laser (4) als Multiline-Singleline-, Multimod- oder als durchstimmbarer Laser ausgebildet ist.

15. Vorrichtung nach einem der vorhergehenden Ansprüche, **dadurch gekennzeichnet,** daß der Laser (4) als Dioden-Laser für Frequenzen von 5 x 10¹⁴ Hz bis 10¹⁵ Hz ausgelegt ist.

16. Vorrichtung nach Anspruch 15, **dadurch gekennzeichnet,** daß der Laser (4) als Kristall-Laser bzw. als Neodym-Yak-Laser ausgelegt ist.

17. Vorrichtung nach Anspruch 1, **dadurch gekennzeichnet,** daß in die Aufnahmevorrichtung (3) zur Behandlung von Prellungen ein Festkörper oder gasförmiges oder flüssiges Gemisch (8) einbringbar ist, das in der Konzentration von 10 x 10⁻³ bis 10 x 10⁻⁹ Vol.-% vorliegt.

18. Vorrichtung nach Anspruch 17, **dadurch gekennzeichnet,** daß in die Aufnahmevorrichtung (3) zur Behandlung von Knochenbrüchen ein Festkörper oder gasförmiges oder flüssiges Gemisch (8) einbringbar ist, das in der Konzentration von 10 x 10⁻³ bis 10 x 10⁻¹² Vol.-% vorliegt, wobei hierzu die Argon-Ion-Laservorrichtung (4) eingesetzt wird.

19. Vorrichtung nach Anspruch 18, **dadurch gekennzeichnet,** daß in die Aufnahmevorrichtung (3) zur Behandlung von Tumoren ein Festkörper oder gasförmiges oder flüssiges Gemisch (8) einbringbar ist, das in dar Konzentration von 10 x 10⁻⁶ bis 10 x 10⁻⁸ Vol.-% vorliegt, wobei hierzu die Argon-Krypton-Mischgas-Laservorrichtung (4) eingesetzt wird.

20. Vorrichtung nach einem der vorhergehenden Ansprüche, **dadurch gekennzeichnet,** daß in die Aufnahmevorrichtung (3) zur Behandlung von Gehirnerschütterungen ein Festkörper oder gasförmiges oder flüssiges Gemisch (8) einbringbar ist, das in der Konzentration von 10 x 10⁻³ bis 10 x 10⁻⁹ Vol.-% vorliegt, wobei hierzu die Argon-Ion-Laservorrichtung (4) eingesetzt wird.

21. Vorrichtung nach einem der vorhergehenden Ansprüche, **dadurch gekennzeichnet,** daß in die Aufnahmevorrichtung (3) zur Behandlung von Stoffwechselerkrankungen ein Festkörper oder gasförmiges oder flüssiges Gemisch (8) einbringbar ist, das in der Konzentration von 10 x 10⁻¹ Vol.-% bis 10 x 10⁻²⁴ Vol.-% vorliegt, wobei hierzu die Argon-Ion- und/oder Argon-Krypton-Mischgas- und/oder Dioden- und/oder Neodym-Yak- und/oder He-Ne- und/oder He-Cd-Laservorrichtung (4) eingesetzt wird.

22. Vorrichtung nach einem der vorhergehenden Ansprüche, **dadurch gekennzeichnet,** daß in die Aufnahmevorrichtung (3) zur Behandlung von Zellkulturen ein Festkörper oder gasförmiges oder flüssiges Gemisch (8) einbringbar ist, das in der Konzentration von 100 Vol.-% bis 10 x 10⁻²⁴ Vol.-% vorliegt, wobei hierzu die Argon-Ion-, Argon-Krypton-, Krypton-, Dioden-, Neodym-Yak-, He-Ne-, He-Cd-Laservorrichtung (4) eingesetzt wird.

23. Vorrichtung nach einem der vorhergehenden Ansprüche, **dadurch gekennzeichnet,** daß das Gerät oder der CD-Player, über das eine Toninformation oder Musikinformation hergestellt, brauchbar gemacht und/oder an eine andere Einrichtung weitergegeben wird, einen oder mehrere Ausgänge (57, 57') aufweist, wobei der eine Ausgang (57) mit der Laservorrichtung (4) und der andere Ausgang (57') mit einem Kopfhörer und/oder mindestens einem Lautsprecher (71) mittel- oder unmittelbar in Verbindung steht.
